(19) Europäisches Patentamt
European Patent Office
Office européen des brevets

(11) EP 4 286 040 A1

(12) **EUROPEAN PATENT APPLICATION**

(43) Date of publication:
**06.12.2023 Bulletin 2023/49**

(21) Application number: **22305808.2**

(22) Date of filing: **02.06.2022**

(51) International Patent Classification (IPC):
*B01J 13/14* (2006.01)   *B01J 13/18* (2006.01)
*A01N 25/28* (2006.01)   *A23P 10/30* (2016.01)
*A61K 8/11* (2006.01)    *A61K 9/50* (2006.01)
*C09B 67/02* (2006.01)   *C11D 3/50* (2006.01)
*F28D 20/02* (2006.01)

(52) Cooperative Patent Classification (CPC):
**B01J 13/185; A01N 25/28; A23P 10/30;
A61K 8/11; B01J 13/14**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Calyxia
94380 Bonneuil-sur-Marne (FR)**

(72) Inventors:
• **NANNETTE, Claire**
  **94380 Bonneuil-sur-Marne (FR)**
• **BAUDRY, Jean**
  **94380 Bonneuil-sur-Marne (FR)**
• **BIBETTE, Jerome**
  **94380 Bonneuil-sur-Marne (FR)**
• **DEMOULIN, Damien**
  **94380 Bonneuil-sur-Marne (FR)**

(74) Representative: **Icosa
83 avenue Denfert-Rochereau
75014 Paris (FR)**

(54) **DOUBLE EMULSION AND CAPSULES**

(57)   The present invention relates to a method for the manufacture of a water-in-oil-in-water double emulsion comprising a step of adding dropwise an aqueous phase to an oil phase until a catastrophic inversion. The present invention also relates to a water-in-oil-in-water double emulsion, a method for preparing solid microcapsules and solid microcapsules.

EP 4 286 040 A1

**Description**

**FIELD OF INVENTION**

The present invention relates to a water-in-oil-in-water double emulsion, a method for the manufacture of a water-in-oil-in-water double emulsion, solid microcapsules and a method for the manufacture of solid microcapsules.

**BACKGROUND OF INVENTION**

Encapsulating and isolating active agents allows the protection thereof from the surrounding environment and in particular from hydrolysis, thermal degradation, oxidation, cross-reactivity or other processes that may reduce their performance.

In the prior art, some encapsulation processes have been described, in particular emulsion techniques. However, the emulsion systems of the prior art require the use of surfactants or emulsifiers to be stabilized, said surfactants and emulsifiers having the disadvantage of being able to react with the encapsulated active agent and/or provide contaminants in the different phases. In addition, surfactants in emulsions cause significant environmental problems, and the successive steps involved in capsule fabrications processes based on emulsion techniques result in significant energy costs and process complexity, which lowers the key performance indicator of yield/time.

Therefore, the present invention aims at providing a method for manufacturing solid microcapsules without using surfactants during the elaboration of emulsions.

However, several constraints can be highlighted when producing double emulsions without using surfactants. Indeed, it is a two-step method (for example described in WO2018172360), during which a first emulsion is made and then re-emulsified in an external phase. On the one hand, it is necessary to ensure the stability of the primary emulsion. On the other hand, it is necessary to concentrate this emulsion sufficiently in the innermost phase in order to increase the core volume of the capsules and thus the encapsulation efficiency.

The encapsulation yield is particularly low, based on techniques disclosed in the prior art, for hydrophilic active agents. Indeed, the continuous phase is generally aqueous and the hydrophilic active tends to disperse in the continuous phase rather than being encapsulated by the oligomeric phase, due to osmotic pressure between the innermost aqueous phase and the external aqueous phase, through the oil middle phase.

Therefore, the present invention aims at providing a method in one step for manufacturing a double emulsion without surfactants and an encapsulation method optimally suited for hydrophilic active agents.

The present inventors have surprisingly discovered a method for manufacturing water-in-oil-in-water double emulsions that have the particular advantage of being able to formulate remarkably stable invert emulsions, said method being in one-step, without the use of a surfactant, allowing the encapsulation of more hydrophilic active agent than the prior art methods (which generally allows the encapsulation of about 5-20% w/w of active agent relative to the total weight of the emulsion). Indeed, phases separation in the emulsion occurs only several hours after the emulsion is formed and left at rest. Moreover, concentrated to a limit fraction $\varphi$max, they invert according to a specific scheme where the initial W/O single emulsion gives rise to a W/O/W double emulsion. In addition, at least 50% w/w of active agent relative to the total weight of the emulsion are encapsulated with the method according to the invention.

**SUMMARY**

The present invention relates to a method for the manufacture of a water-in-oil-in-water double emulsion, comprising:

a step of adding dropwise an aqueous phase to an oil phase, wherein the adhesion energy between two droplets of aqueous phase dispersed in the oil phase ranges from $10^{-5}$ J.m$^{-2}$ to $10^{-3}$ J.m$^{-2}$, said step of adding the aqueous phase to the oil phase being performed until a catastrophic inversion occurs.

Advantageously, the addition dropwise of the aqueous phase to the oil phase occurs at a rate ranging from 0.001 mL.s$^{-1}$ to 50 mL.s$^{-1}$, preferably from 0.001 mL.s$^{-1}$ to 20 mL.s$^{-1}$, more preferably from 0.001 mL.s$^{-1}$ to 10 mL.s$^{-1}$, even more preferably from 0.001 mL.s$^{-1}$ to 0.2 mL.s$^{-1}$, better from 0.001 mL.s$^{-1}$ to 0.05 mL.s$^{-1}$, still better of about 0.01 mL.s$^{-1}$.

Advantageously, the mixture comprising the aqueous phase and the oil phase is continuously stirred, preferably at a stirring speed ranging from 100 rpm to 3000 rpm, more preferably at a stirring speed ranging from 500 rpm to 2500 rpm. More advantageously, the shear rate applied during stirring ranges from 1000 s$^{-1}$ to 4000 s$^{-1}$, preferably from 2000 s$^{-1}$ to 3000 s$^{-1}$.

Advantageously, said double emulsion does not comprise surfactant.

Advantageously, the ratio between the dynamic viscosity of the innermost aqueous phase and the viscosity of the oil phase ranges from 0.01 to 20, preferably from 0.01 to 10, more preferably from 0.1 to 10, even more preferably from 1 to 10. The dynamic viscosities may in particular be measured using a TA Instruments AR-G2 rheometer equipped with a 3 cm diameter, 2-degree angle cone and a temperature control cell set at 25°C.

Advantageously, the interfacial tension $\gamma$ between the aqueous phase and the oil phase ranges from 0 J.m$^{-2}$ to $50\times10^{-3}$ J.m$^{-2}$, preferably from $20\times10^{-3}$ J.m$^{-2}$ to $40\times10^{-3}$ J.m$^{-2}$.

Advantageously, the oil phase comprises at least one compound selected from oligomers, monomers and mixtures thereof, and optionally at least one photo initiator.

The present invention also relates to a water-in-oil-in-water double emulsion obtained by the method according to the invention for the manufacture of a water-in-oil-in-water double emulsion.

The present invention also relates to a water-in-oil-in-water double emulsion, comprising two aqueous phases (an innermost and an external aqueous phases) and one oil phase, wherein one of the two aqueous phases (the innermost aqueous phase) is entrapped in the oil phase and the second aqueous phase (the external aqueous phase) entraps the oil phase, wherein the two aqueous phases comprise the same composition, wherein the adhesion energy between two droplets of the innermost aqueous phase dispersed in the oil phase ranges from $10^{-5}$ J.m$^{-2}$ to $10^{-3}$ J.m$^{-2}$.

Advantageously, said double emulsion does not comprise surfactant.

Advantageously, the ratio between the dynamic viscosity of the innermost aqueous phase and the viscosity of the oil phase ranges from 0.01 to 20, preferably from 0.01 to 10, more preferably from 0.1 to 10, even more preferably from 1 to 10. The dynamic viscosities may in particular be measured using a TA Instruments AR-G2 rheometer equipped with a 3 cm diameter, 2-degree angle cone and a temperature control cell set at 25°C.

Advantageously, the interfacial tension $\gamma$ between the innermost aqueous phase and the oil phase ranges from 0 J.m$^{-2}$ to $50\times10^{-3}$ J.m$^{-2}$, preferably from $20\times10^{-3}$ J.m$^{-2}$ to $40\times10^{-3}$ J.m$^{-2}$.

Advantageously, the oil phase comprises at least one compound selected from oligomers, monomers and mixtures thereof, and optionally at least one photo initiator.

The present invention also relates to a method for preparing solid microcapsules comprising the steps of:

a) manufacturing a water-in-oil-in-water double emulsion according to the method according to the invention for the manufacture of a water-in-oil-in-water double emulsion, wherein the oil phase comprises at least one compound selected from oligomers, monomers and mixtures thereof and optionally at least one photo initiator, and then

b) crosslinking the oligomers and/or monomers of the oil phase.

Advantageously, the step b) of crosslinking is carried out by submitting the double emulsion to a source of light, preferably a source of UV light.

The present invention also relates to a solid microcapsule obtained by the method according to the invention for preparing solid microcapsules.

The present invention further relates to a solid microcapsule comprising:

- a polymer that is crosslinked in three dimensions forming a polymer network, the polymer network delimiting the capsule shell and creating a matrix in the core of the capsule, and
- drops of an aqueous phase entrapped in the polymer matrix in the core of the capsule.

Advantageously, the aqueous phase comprises at least one hydrophile active agent. Preferably, said solid microcapsule comprises greater than or equal to 20% by weight, more preferably from 20% to 80% by weight, even more preferably from 40% to 60% by weight, better about 50% by weight, of at least one hydrophile active agent, the weight percentages being expressed relative to the total weight of said solid microcapsule.

Advantageously, the ratio of the weight of the entrapped aqueous phase to the weight of the polymer network ranges from 20 to 70.

**DEFINITIONS**

In the present invention, the following terms have the following meanings:

**"About",** before a figure or number, refers to plus or minus 10% of the face value of that figure or number. In one embodiment, "about", before a figure or number, refers to plus or minus 5% of the face value of that figure or number.

**"Active agent"** or **"active ingredient"** refers to a substance that has an effect, in particular in a field selected from the group consisting of therapeutic, cosmetic, agriculture, home care, chemical, paint, fuel, lubricant, bitumen, and drilling sludges or muds. The agent may be a chemical or a biological substance. Preferably, the active agent is a chemical substance.

Advantageously, the active agent is selected from the group consisting of:

- a crosslinker, hardener, curing agent (such as a urea derivative), curing agent accelerator (such as amide, amine and imidazole derivatives), organic or metallic catalyst (such as an organometallic or inorganometallic complex of platinum, palladium, titanium, molybdenum, copper, zinc or salts of metallic complexes dissolved in polyethylene glycol or polyethylene glycol derivatives) used to polymerize polymer, elastomer, rubber, paint, adhesive, sealant, mortar, varnish or coating formulations;
- a colorant or pigment for use in elastomer, paint, coating, adhesive, sealant, mortar, or paper formulations;
- a fragrance (as defined by the International Fragrance Association (IFRA) list of molecules available at www.ifraorg.org) for use in detergents such as laundry detergents, home care products, cosmetics and personal care products, textiles, paints, coatings;
- a flavouring, a vitamin, an amino acid, a protein, a lipid, a probiotic, an antioxidant, a pH corrector, a preservative for food compounds and animal feed;
- a plant extract or an essential oil derivative such as propolis extract, menthol derivatives or poppy petal extract for personal care formulations;
- a softener, a moisturizer, a conditioner for detergents, laundry, cosmetics and personal care products. In this respect, the active ingredients that can be used are, for example, listed in patents US 6 335 315 and US 5 877 145;
- an anti-color alteration agent (such as an ammonium derivative), an anti-foaming agent (such as an alcohol ethoxylate, an alkylbenzene sulfonate, a polyethylene ethoxylate, an alkyl ethoxysulfate or an alkyl sulfate) for detergents and laundry and home care products;
- a brightening agent, also known as a color activator (such as a stilbene derivative, a coumarin derivative, a pyrazoline derivative, a benzoxazole derivative or a naphthalimide derivative) for use in detergents, laundry products, cosmetics and personal care products;
- a biologically active compound such as an enzyme, a vitamin, a protein, a plant extract, an emollient agent, a disinfectant agent, an antibacterial agent, an anti-UV agent, a drug intended for cosmetic and personal care products, and textiles. Among these biologically active compounds we can mention : vitamins A, B, C, D and E, para aminobenzoic acid, alpha hydroxy acids (such as glycolic acid, lactic acid, malic acid, tartaric acid or citric acid), camphor, ceramides, polyphenols (such as flavonoids, phenolic acid, ellagic acid, tocopherol, ubiquinol), hydroquinone, hyaluronic acid, isopropyl isostearate, isopropyl palmitate, oxybenzone, panthenol, proline, retinol, retinyl palmitate, salicylic acid, sorbic acid, sorbitol, triclosan, tyrosine;
- a disinfectant, an antibacterial agent, an anti-UV agent, for paints and coatings;
- a fertilizer, herbicide, insecticide, pesticide, fungicide, repellent or disinfectant for agrochemicals;
- a flame retardant (such as a brominated polyol like tetrabromobisphenol A, a halogenated or non-halogenated organophosphorus compound, a chlorine compound, an ammonium polyphosphate, an aluminum trihydrate, an antimony oxide, a zinc borate, a red phosphorus, a melamine, or a magnesium dihydroxide) for use in plastics, coatings, paints and textiles;
- a photonic crystal or photochromophore for use in paints, coatings and polymeric materials forming curved and flexible displays; and
- a Phase Change Materials (PCM) capable of absorbing or releasing heat when undergoing a phase change, intended for energy storage. Examples of PCMs include molten aluminum phosphate, ammonium carbonate, ammonium chloride, cesium carbonate, cesium sulfate, calcium citrate, calcium chloride, calcium hydroxide, calcium oxide, calcium phosphate, calcium saccharate, calcium sulfate, cerium phosphate, iron phosphate, lithium carbonate, lithium sulfate, magnesium chloride, magnesium sulfate, manganese chloride, manganese nitrate, manganese sulfate, potassium acetate, potassium carbonate, potassium chloride, potassium phosphate, rubidium carbonate, rubidium sulfate, disodium tetraborate, sodium acetate, sodium bicarbonate, sodium bisulfate, sodium citrate, sodium chloride, sodium hydroxide, sodium nitrate, sodium percarbonate, sodium persulfate sodium phosphate, sodium propionate, sodium selenite, sodium silicate, sodium sulfate, sodium tellurate, sodium thiosulfate, strontium hydrophosphate, zinc acetate, zinc chloride, sodium thiosulfate, paraffinic hydrocarbon waxes, and polyethylene glycols.

Advantageously, the active agent is hydrophile. More advantageously, the active agent is selected from the group consisting of:

- a hydrophile crosslinker, hardener, curing agent accelerator (such as amide, amine and imidazole derivatives), or a hydrophile metallic catalyst such as salts of metallic complexes dissolved in polyethylene glycol or polyethylene glycol derivatives used to polymerize polymer, elastomer, rubber, paint, adhesive, sealant, mortar, varnish or coating formulations;
- a hydrophile colorant or pigment for use in elastomer, paint, coating, adhesive, sealant, mortar, or paper formulations;
- a hydrophile flavouring, vitamin, amino acid, protein, probiotic, antioxidant, pH corrector, preservative for food compounds and animal feed;
- a hydrophile plant extract or essential oil derivative such as propolis extract, menthol derivatives or poppy petal extract for personal care formulations;

- a hydrophile softener, conditioner for detergents, laundry, cosmetics and personal care products selected from amine derivatives;
- a hydrophile biologically active compound selected from hydrophile enzymes, vitamins, proteins, antibacterial agents.
- a hydrophile fertilizer, herbicide, insecticide, pesticide, fungicide, repellent or disinfectant for agrochemicals;
- a hydrophile flame retardant such as a chlorine compound or an ammonium polyphosphate, for use in plastics, coatings, paints and textiles;
- a hydrophile Phase Change Materials (PCM) such as ammonium carbonate, ammonium chloride, calcium chloride, calcium hydroxide, calcium phosphate, calcium saccharate, calcium sulfate, magnesium chloride, magnesium sulfate, manganese chloride, manganese nitrate, manganese sulfate, potassium acetate, potassium carbonate, potassium chloride, potassium phosphate, sodium acetate, sodium bicarbonate, sodium bisulfate, sodium chloride, sodium hydroxide, sodium nitrate, sodium percarbonate, sodium persulfate, sodium phosphate, sodium propionate, sodium sulfate, sodium tellurate, sodium thiosulfate, zinc acetate, zinc chloride, sodium thiosulfate, and polyethylene glycols.

**"Additional monomers or oligomers"** refers to monomers or oligomers bearing at least one pH sensitive group, temperature sensitive group, ultraviolet (UV) sensitive group and/or infrared (IR) sensitive group. These additional monomers or oligomers can induce the rupture of the solid microcapsules according to the invention and subsequently the release of their contents, after stimulation via change in pH or temperature, or exposure to UV or IR radiation. Advantageously, these additional monomers or oligomers may be selected from the group consisting of those:

- bearing at least one reactive function selected from the group consisting of acrylate, alcohol acrylates, methacrylate, vinyl ether, N-vinyl ether, mercaptoester, thiolene, silane, siloxane, epoxy, oxetane, urethane, isocyanate and peroxide functions, and also
- bearing one of the following groups:

o a hydrophobic group such as a fluorinated group, for example trifluoroethyl methacrylate, trifluoroethyl acrylate, tetrafluoropropyl methacrylate, pentafluoropropyl acrylate, hexafluorobutyl acrylate, or fluorophenyl isocyanate;
o a pH-sensitive group such as primary amines, secondary amines, tertiary amines, carboxylic acids, phosphate, sulfate, nitrate, or carbonate groups;
o a UV-sensitive or UV-cleavable group (or photochromic group) such as azobenzene, spiropyran, 2-diazo-1,2-naphthoquinone, o-nitrobenzyl, thiol, or 6-nitro-veratroyloxycarbonyl groups, e.g., poly(ethylene oxide)-block-poly(2-nitrobenzylmethacrylate), and other block copolymers, as described in particular in Liu et al, Polymer Chemistry 2013, 4, 3431-3443;
o an IR-sensitive or IR-cleavable group such as o-nitrobenzyl or 2-diazo-1,2-naphthoquinone, e.g., the oligomers described in Liu et al., Polymer Chemistry 2013, 4, 3431-3443; and
o a temperature sensitive group such as poly(N-isopropylacrylamide).

Advantageously, these additional monomers or oligomers may be selected from the group consisting of silanes and alcohol acrylates.

**"Adhesion"** refers to the ability of suspended particles or droplets to stick together when approaching closely. When two droplets come into close contact, their surface can deform resulting in the occurrence of a contact angle $\theta$ between them. Adhesion can thus be also described as "wetting". Adhesion takes place into systems where there exists an attractive interaction between both O/W interfaces. This interaction can be attributed to intermolecular forces, chain entanglements, or both, across the interfaces. Adhesion in a system of aqueous droplets suspended in an external oil phase can be quantitatively characterized through the measurement of an **"adhesion energy"**. The adhesion energy may be measured by any method known by the person skilled in the art. There are many available methods for measurement of adhesion energy and the resultant value is independent from the measurement method. For example, the adhesion energy may be measured by a method selected from the group consisting of the method described in Poulin et al, Phys. Rev. Lett. 77:3248, 1996, the method described in Poulin et al, Phys. Rev. Lett. 79:4862, 1997, and the adhesion test method (consisting in using an adhesion test with a micropipette aspiration and the formula: $E = 2\gamma(1 - \cos\theta)$). Advantageously, the energy adhesion E may be calculated by the formula: $E = 2\gamma(1 - \cos\theta)$, wherein $\gamma$ is the interfacial tension between the oil and the aqueous phase and $\theta$ is the contact angle between the droplets. $\theta$ can be measured through different techniques depending on its value. When $\theta > 30°$, it can be obtained through direct microscopic measurement and image analysis techniques with a geometrical estimation. When $\theta < 30°$, it is obtained through the observation of the oil film between the adhesive droplets through the formula

$$2\theta = sin^{-1}\left(\frac{R_{film}}{R_{g1}}\right) + sin^{-1}\left(\frac{R_{film}}{R_{g2}}\right)$$

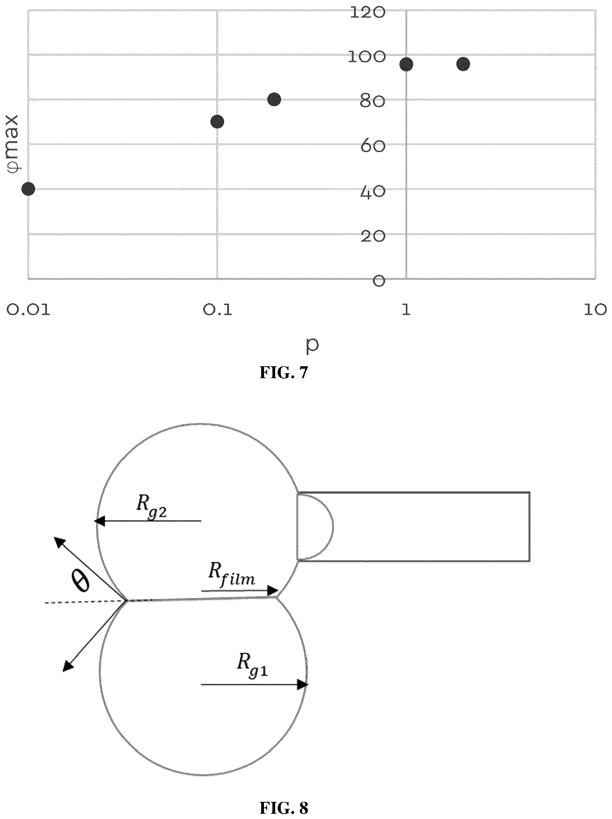

, wherein $R_{film}$ is the radius of the oil film and $R_{g1,2}$ are the radius of the drops. In that particular case, owing to its low reflectivity, the film can be observed under optical or confocal microscope. **"Adhesion test with a micropipette aspiration"** refers to one exemplary method of determining the adhesion between two drops, the method comprising the use of a micropipette aspiration for adhesion testing as represented in **Figure 7,** allowing the precise determination of the oil film size using a confocal microscope.

**"Aqueous phase"** refers to a solution comprising a solvent and optionally at least one solute, wherein said solvent is selected from the group consisting of water, hydrophilic organic solvents and combinations thereof. According to the invention, the aqueous phase and the oil phase are immiscible in each other, which means that the amount by weight of the aqueous phase capable of being solubilized in the oil phase is less than or equal to 5%, preferably less than 1%, more preferably less than 0, 5%, even preferably of 0%, based on the total weight of the oil phase, and that the amount (by weight) of the oil phase capable of being solubilized in the aqueous phase is less than or equal to 5%, preferably less than 1%, more preferably less than 0.5%, even preferably of 0%, based on the total weight of the aqueous phase. Advantageously, the immiscibility between the aqueous phase and the oil phase makes it possible to avoid migration of active agent(s) optionally present in the aqueous phase from said aqueous phase to the oil phase.

**"Catastrophic inversion"** or **"phase inversion"** or **"continuity inversion"** refers to a change in the mutual dispersion of two phases in contact in an emulsion. For example, the catastrophic inversion may allow the change of a W/O simple emulsion to a O/W simple emulsion or to a W/O/W double emulsion. Advantageously, the catastrophic inversion allows the change of a W/O emulsion to a W/O/W emulsion.

**"Comprising"** or **"comprise"** is to be construed in an open, inclusive sense, but not limited to. In an embodiment, "comprising" means "consisting essentially of". In an embodiment, "comprising" means "consisting of', which is to be construed as limited to.

**"Crosslinkable"** refers to a composition capable of polymerizing (crosslinking) to give a solid material, to form the polymerized shell and network of solid microcapsules according to the invention.

**"Crosslinking agent"** refers to a compound carrying at least two reactive functions capable of crosslinking a monomer or an oligomer, or a mixture of monomers or oligomers, during its polymerization. Advantageously, the crosslinking agent may be selected from molecules carrying at least two functions selected from the group consisting of acrylate, methacrylate, vinyl ether, N-vinyl ether, mercaptoester, thiolene, siloxane, epoxy, oxetane, urethane, isocyanate and peroxide functions. Advantageously, the crosslinking agent may be selected from the group consisting of:

- diacrylates, such as 1,6-hexanediol diacrylate, 1,6-hexanediol dimethacrylate, polyethylene glycol dimethacrylate, 1,9-nonanediol dimethacrylate, 1,4-butanediol dimethacrylate, 2,2-bis(4-methacryloxyphenyl) propane, 1,3-butanediol dimethacrylate, 1,10-decanediol dimethacrylate, bis(2-methacryloxyethyl) N,N'-1,9-nonylene biscarbamate, 1,4-butanediol diacrylate, ethylene glycol diacrylate, 1,5-pentanediol dimethacrylate, 1,4-Phenylene diacrylate, allyl methacrylate, N,N'-methylenebisacrylamide, 2,2-bis[4-(2-hydroxy-3-methacryloxypropoxy)phenyl]propane, tetraethylene glycol diacrylate, ethylene glycol dimethacrylate, diethylene glycol diacrylate triethylene glycol diacrylate, triethylene glycol dimethacrylate, polyethylene glycol diglycidyl ether, N,N-diallylacrylamide, 2,2-bis[4-(2-acryloxyethoxy) phenyl]propane, glycidyl methacrylate;
- multifunctional acrylates such as dipentaerythritol pentaacrylate, 1,1,1-trimethylolpropane triacrylate, 1,1,1-trimethylolpropane trimethacrylate, ethylenediamine tetramethacrylate, pentaerythritol triacrylate, pentaerythritol tetraacrylate; and
- acrylates also having another reactive function, such as propargyl methacrylate, 2-Cyanoethyl acrylate, tricyclodecane dimethanol diacrylate, hydroxypropyl methacrylate, N-acryloxysuccinimide, N-(2-Hydroxypropyl)methacrylamide, N-(3-aminopropyl)methacrylamide hydrochloride, N-(t-BOC-aminopropyl)methacrylamide 2-aminoethyl methacrylate hydrochloride, monoacryloxyethyl phosphate, o-nitrobenzyl methacrylate, acrylic anhydride, 2-(tert-butylamino)ethyl methacrylate, N, N-diallylacrylamide, glycidyl methacrylate, 2-hydroxyethyl acrylate, 4-(2-acryloxyaethoxy)-2-hydroxybenzophenone, N-(Phthalimidomethyl)acrylamide, cinnamyl methacrylate.

**"Drop"** refers to a small liquid particle. **"Single drop"** refers to a drop consisting of a single drop phase. **"Double drop"** refers to a drop comprising two phases in the drop, organized in the form of at least one internal drop (composed of one phase) surrounded by an external drop (composed of the other phase).

**"Emulsion"** refers to a fluid colloidal system in which liquid droplets are dispersed in a liquid. The droplets often exceed the usual limits for colloids in size. A **"simple emulsion"** is denoted by the symbol O/W (or by the term oil-in-water) if the continuous phase is an aqueous solution (=aqueous phase) and by W/O (or by the term water-in-oil) if the continuous phase is an organic liquid (an "oil"). **"Double emulsions"** are more complicated emulsion, such as W/O/W (also named water-in-oil-in-water double emulsion, i.e. aqueous droplets contained within oil droplets dispersed

6

in a continuous aqueous phase). In a W(1)/O/W(2) double emulsion, the internal emulsion refers to the emulsion of the innermost aqueous phase W(1) in the oil phase O; and the external emulsion refers to the emulsion of the oil phase O in the external aqueous phase W(2).

**"From X to Y"** refers to the range of values between X and Y, the limits X and Y being included in said range.

**"Hydrophilic"** or **"hydrophile"** refers to the capacity of a molecular entity to interact with polar solvents, in particular with water.

**"Inclusions"** refers to spaces in a polymer (the host), in which molecular entities of a second chemical species (the guest) are located. In particular, said spaces may be in the shape of long tunnels, channels, or hollow (in particular hollow shaped like a ball or like a drop). The spaces in the polymer lattice are enclosed on all sides so that the guest species is 'entrapped' as in a cage. Preferably, there is no covalent bonding between guest and host. Advantageously, the guest is the aqueous phase according to the invention. Advantageously, the polymer host is a polymer issued from the polymerization of the oil phase according to the invention.

**"Initiator"** refers to any substance introduced in the oil phase in order to bring about a polymerization reaction when said initiator is excited by a source of energy. **"Photoinitiator"** refers to any substance introduced in the oil phase in order to bring about a polymerization reaction when said initiator is excited by a light radiation. Advantageously, the photoinitiators may be selected from the group consisting of:

- $\alpha$-hydroxyketones, such as 2-hydroxy-2-methyl-1-phenyl-1-propanone;
- $\alpha$-aminoketones, in particular 2-benzyl-2-dimethylamino-1-(4-morpholinophenyl)-butanone-1;
- aromatic ketones or thioxanthones, and quinones. These aromatic ketones most often require the presence of a hydrogen donor compound such as tertiary amines and especially alkanolamines;
- the $\alpha$-dicarbonyl derivatives of which the most common representative is benzyldimethylketal;
- acylphosphine oxides, such as, for example, bis-acylphosphine oxides (BAPO); and
- other photoinitiators including aromatic ketones such as benzophenone, phenylglyoxylates such as phenylglyoxylic acid methyl ester, oxime esters such as [1-(4-phenylsulfanylbenzoyl)heptylideneamino]benzoate, sulfonium salts, iodonium salts and oxime sulfonates.

**"Inverting emulsion"** or **"inverse emulsion"** refers to an emulsion in which the mutual dispersion of two phases in contact with each other has changed.

**"Mean diameter"** refers to the $Dn_{50}$ diameter. $Dn_{50}$ is the median of the number averaged size distribution of the droplets or (micro)capsules. The size distribution of the droplets or (micro)capsules, and thus the mean diameter of the droplets or (micro)capsules, may be measured by methods well known to the skilled person in the art, e.g. by a light scattering technique (for example using a Mastersizer 3000 equipped with a hydro SV measuring cell), or by image analysis of optical microscopy pictures, or by image analysis of electronic microscopy pictures (TEM - Transmission electron microscopy).

**"Metastability"** refers to the state of a phase in which an energy barrier considerably higher than $k\,T$ must be overcome before this phase can transform to a phase of lower molar Gibbs energy and molar Helmholtz energy, where k is the Boltzmann constant ($k$=1.380649$\times$10$^{-23}$ J.K$^{-1}$) and T the thermodynamic temperature.

**"Monomer"** refers to a molecule which can undergo polymerization thereby contributing constitutional units to the essential structure of a macromolecule. **"Oligomer"** refers to a macromolecule comprising a repetition of monomers, preferably a repetition of less than 10 monomers. Advantageously, the **"monomers or oligomers"** may be selected from monomers and oligomers comprising at least one reactive function selected from the group consisting of acrylate, methacrylate, vinyl ether, N-vinyl ether, mercaptoester, thiolene, siloxane, epoxy, oxetane, urethane, isocyanate and peroxide functions. More advantageously, the monomers or oligomers may be selected from the group consisting of aliphatic or aromatic esters or polyesters, urethanes or polyurethanes, anhydrides or polyanhydrides, saccharides or polysaccharides, ethers or polyethers, amides or polyamides, and carbonates or polycarbonates, wherein the monomers or oligomers further include at least one reactive moiety selected from the group consisting of acrylate, methacrylate, vinyl ether, N-vinyl ether, vinyl ester, thiolene, maleate, epoxy, siloxane, amine, lactone, phosphate, and carboxylate moieties. Even more advantageously, the monomers or oligomers may be selected from monomers or oligomers bearing at least one of the above-mentioned reactive functions and additionally bearing at least one function selected from the group consisting of primary, secondary and tertiary alkylamine functions, quaternary amine functions, sulfate, sulfonate, phosphate, phosphonate, carboxylate, hydroxyl, halogen functions and mixtures thereof.

**"Oil phase"** refers to a phase not miscible with an aqueous phase, which means that the amount by weight of an aqueous phase capable of being solubilized in the oil phase is less than or equal to 5%, preferably less than 1%, more preferably less than 0, 5%, even preferably of 0%, based on the total weight of the oil phase, and that the amount (by weight) of the oil phase capable of being solubilized in an aqueous phase is less than or equal to 5%, preferably less than 1%, more preferably less than 0.5%, even preferably of 0%, based on the total weight of the aqueous phase. The oil phase does not necessarily contain oil. The phase oil has typically a viscosity greater than the water viscosity.

Advantageously, the oil phase comprises oligomers, monomers and optionally initiators (such as photoinitiators). More advantageously, the oil phase may further include at least one oil. Even more advantageously, the phase oil may further comprise at least one oil selected from the group consisting of silicone oils, mineral oils and mixtures thereof. In particular, the silicone oils may be selected from the group consisting of poly(dimethylsiloxane) (PDMS), poly(monomethylsiloxane) (PPMS), poly(dimethylsiloxane) hydroxy terminated (PDMS-OH) and mixtures thereof. In particular, the mineral oils may be selected from the group consisting of soybean oil, sunflower oil, jojoba oil, coconut oil, lanolin oil, castor oil, derivatives thereof and mixtures thereof.

**"Polymer"** refers to a substance composed of macromolecules, said macromolecules comprising multiple repetition of monomers. Advantageously, the polymers may be selected from the group consisting of polyethers, polyepoxides, polyesters, polyurethanes, polyureas, polyethylene glycols, polypropylene glycols, polyamides, polyacetals, polyimides, polyolefins, polysulfides and polydimethylsiloxanes. More advantageously, the polymer may be selected from the group consisting of polyesters, polyepoxides, and polyurethanes. Even more advantageously, said polymers further carry at least one reactive functionality selected from the group consisting of acrylate, methacrylate, vinyl ether, N-vinyl ether, mercaptoester, thiolene, siloxane, epoxy, oxetane, urethane, isocyanate, and peroxide functionality. For example, the polymers may be selected from the group consisting of: poly(2-(1-naphthyloxy)-ethyl acrylate), poly(2-(2-naphthyloxy)-ethyl acrylate), poly(2-(2-naphthyloxy)-ethyl methacrylate), polysorbitol dimethacrylate, polyacrylamide, poly((2-(1-naphthyloxy) ethanol), poly(2-(2-naphthyloxy) ethanol), poly(1-chloro-2,3-epoxypropane), poly(n-butyl isocyanate), poly(N- vinyl carbazole), poly(N-vinyl pyrrolidone), poly(p-benzamide), poly(p-chlorostyrene), poly(p-methyl styrene), poly(p-phenylene oxide), poly(p-phenylene sulfide), poly(N-(methacryloxyethyl)succinimide), polybenzimidazole, polybutadiene, polybutylene terephthalate, polychloral, polychloro trifluoro ethylene polyether imide, polyether ketone, polyether sulfone, polyhydridosilsesquioxane, poly(m-phenylene isophthalamide), poly(methyl 2-acrylamido-2-methoxyaceate), poly(2-acrylamido-2-methylpropanesulfonic acid), poly-mono-butyl maleate, polybutylmethacrylate, poly(N-tert-butylmethacrylamide), poly(N-n-butylmethacrylamide), polycyclohexylmethacrylamide, poly(m-xylenebisacrylamide 2,3-dimethyl-1,3-butadiene, N,N-dimethylmethacrylamide), poly(n-butyl methacrylate), poly(cyclohexyl methacrylate), polyisobutyl methacrylate, poly(4-cyclohexylstyrene), polycyclol acrylate, polycyclol methacrylate, polydiethyl ethoxymethylenemalonate, poly(2,2,2-trifluoroethyl methacrylate), poly(1,1,1-trimethylolpropane trimethacrylate), polymethacrylate, poly(N,N-dimethylaniline, dihydrazide), poly(isophthalic dihydrazine) polyisophthalic acid, polydimethyl benzilketal, epichlorohydrin, poly(ethyl-3,3-diethoxyacrylate), poly(ethyl-3,3-dimethylacrylate), poly(ethyl vinylketone) poly(penten-3-one), polyformaldehyde poly(diallyl acetal), polyfumaronitrile, polyglyceryl propoxy triacrylate, polyglyceryl trimethacrylate, polyglycidoxypropyltrimethoxysilane, polyglycidyl acrylate poly(n-heptyl acrylate), poly(n-heptyl acrylic acid ester), poly(n-heptyl methacrylate), poly(3-hydroxypropionitrile), poly(2-hydroxypropyl acrylate) poly(N-(methacryloxyethyl)phthalimide), poly(1,9-nonanediol diacrylate), poly(1,9-nonanediol dimethacrylate), poly(N-(n-propyl) acrylamide), poly(ortho-phthalic acid), poly(isophthalic acid), poly(1, 4-benzenedicarboxylic acid), poly(1,3-benzenedicarboxylic acid), poly(phthalic acid), poly(mono-2-acryloxyethyl ester), polyterephthalic acid, polyphthalic anhydride, polyethylene glycol diacrylate, polyethylene glycol methacrylate, polyethylene glycol dimethacrylate, poly(isopropyl acrylate), polysorbitol pentaacrylate, polyvinyl bromoacetate, polychloroprene, poly(di-n-hexyl silylene) poly(di-n-propyl siloxane), polydimethyl silylene, polydiphenyl siloxane, polyvinyl propionate, polyvinyl triacetoxysilane, polyvinyl tris-tert-butoxysilane, polyvinyl butyral, polyvinyl alcohol, polyvinyl acetate polyethylene co-vinyl acetate, poly(bisphenol-A polysulfone), poly(1,3-dioxepane), poly(1,3-dioxolane), poly(1,4-phenylene vinylene), poly(2,6-dimethyl-1A-phenylene oxide), poly(4-hydroxybenzoic acid) poly(4-methyl pentene-1), poly(4-vinyl pyridine), polymethylacrylonitrile, polymethylphenylsiloxane, polymethylsilmethylene, polymethylsilsesquioxane, poly(phenylsilsesquioxane), poly(pyromellitimide-1. 4-diphenyl ether), polytetrahydrofuran, polythiophene, poly(trimethylene oxide), polyacrylonitrile, polyether sulfone, polyethylene-co-vinyl acetate, poly(perfluoroethylene propylene), poly(perfluoroalkoxy alkane and poly(styrene-acrylonitrile).

Advantageously, the polymer may have a molecular weight greater than 5000 g.mol$^{-1}$, preferably from 10,000 g.mol$^{-1}$ to 500,000 g.mol$^{-1}$, more preferably from 50,000 g.mol$^{-1}$ to 300,000 g.mol$^{-1}$.

**"Branched polymer"** refers to a polymer having at least one branching point between its two end groups, a branching point being a point of a chain to which is attached a side chain also called branch or hanging chain. A branched polymer may in particular be selected from the group consisting of grafted, comb or star polymers and dendrimers.

**"Polymerization"** refers to the process of converting a monomer mixture of monomers into a polymer.

**"Rpm"** or **"revolutions per minute"** refers to the number of turns in one minute.

**"Shear rate Y"** refers to the velocity gradient in a flowing fluid. The **"shear stress $\sigma$"** applied to an emulsion drop is defined as the tangential force per unit drop area resulting from the macroscopic shear applied to the emulsion during its agitation. The shear stress $\sigma$ (expressed in Pa), the viscosity $\eta$ (expressed in Pa.s) and the shear rate Y (expressed in s$^{-1}$) applied to an emulsion during its agitation are related by the following equation: $\sigma = \eta Y$.

**"Surface tension $\gamma$"** refers to the work required to increase a surface area divided by that area. When two phases are studied it is called **"interfacial tension $\gamma$";** "interfacial tension $\gamma$" is the change in Gibbs energy per unit change in interfacial area for substances in physical contact.

**"Surfactant"** refers to a substance which lowers the surface tension of the medium in which it is dissolved, and/or the interfacial tension with other phases, and, accordingly, is positively adsorbed at the liquid/vapour, liquid/liquid and/or at other interfaces.

**"Viscosity"** or **"dynamic viscosity"** refers, for a laminar flow of a fluid, to the ratio of the shear stress to the velocity gradient perpendicular to the plane of shear. Viscosity may be measured by methods well-known to a skilled person in the art. Unless otherwise indicated, viscosity, in the present application, is measured by any viscosity measurement method well known to person skilled in the art. In particular, viscosity may be measured using a TA Instruments AR-G2 rheometer equipped with a 3 cm diameter, 2-degree angle cone with a plate geometry and a temperature control cell set at 25°C, the viscosity value being read at a shear rate ranging from $0.1$ $s^{-1}$ to $1000$ $s^{-1}$, preferably at a shear rate equal to $10$ $s^{-1}$.

**"Viscosity ratio p"**, **"dynamic viscosity ratio p"** or **"p"** refers to the ratio of the dynamic viscosity of the

$$p = \frac{\eta disp}{\eta cont}$$

dispersed phase to that of the continuous phase: wherein $\eta_{disp}$ is the viscosity of the dispersed phase and $\eta_{cont}$ is the viscosity of the continuous phase.

In the case of a simple emulsion, **"$\varphi$"** is the w/w percentage of the dispersed phase incorporated into the simple emulsion, relative to the total weight of the simple emulsion. In the case of a double emulsion, **"$\varphi$int"** is the w/w percentage of the innermost dispersed phase incorporated into the middle phase, relative to the total weight of said double emulsion; for example, in a water(1)-in-oil-in-water(2) double emulsion, "$\varphi$int" is the w/w percentage of the water(1) phase incorporated into the oil phase, relative to the total weight of said water(1)-in-oil-in-water(2) double emulsion.

**"$\varphi$max"** is the maximum w/w percentage of the dispersed phase incorporated into a simple emulsion, before noticing the phase inversion of the emulsion, relative to the total weight of the emulsion. For example, in a water-in-oil emulsion, "$\varphi$max" is the w/w percentage of the aqueous phase incorporated into the oil phase, before noticing the phase inversion of the emulsion. According to the present invention, cpmax is preferably equal to or greater than 40% w/w relative to the total weight of the W/O simple emulsion, more preferably cpmax ranges from 40% w/w to 95% w/w relative to the total weight of the W/O simple emulsion. An **"elevated $\varphi$max"** is equal to or greater than 64% w/w relative to the total weight of the emulsion. An emulsion having a cpmax equal to or greater than 40% w/w, preferably equal to or greater than 64% w/w, relative to the total weight of the emulsion is a **"high internal phase emulsion".**

## DETAILED DESCRIPTION

### Method for preparing a double emulsion

The present invention relates to a method for the manufacture of a water-in-oil-in-water double emulsion, comprising a step of adding dropwise an aqueous phase to an oil phase until a catastrophic inversion occurs. Advantageously, said method consists in the step of adding dropwise an aqueous phase to an oil phase until a catastrophic inversion.

According to said method, the adhesion energy between two droplets of aqueous phase dispersed in the oil phase ranges from $10^{-5}$ $J.m^{-2}$ to $10^{-3}$ $J.m^{-2}$. The inventors of the present invention have surprisingly discovered that such a range of adhesion energy between two droplets of aqueous phase dispersed in the oil phase allows the formation of a water-in-oil-in-water double emulsion when the catastrophic inversion occurs, without adding surfactants and in only one step.

The method of the invention has the advantage that no surfactant is required for manufacturing said double emulsion.

Advantageously, the method according to the invention for manufacturing W/O/W double emulsions is a tunable process (choice of phases, size of internal and intermediate droplets, fraction of internal phase) and may be adapted to the W/O system used and depending on the final application.

The method according to the invention for manufacturing W/O/W double emulsions allows the formulation of surfactant-free, highly concentrated (high internal phase ratio) and metastable emulsions.

### Addition and agitation speed

The aqueous phase is added dropwise to the oil phase. Advantageously, the aqueous phase is added dropwise to the oil phase at a rate ranging from $0.001$ $mL.s^{-1}$ to $50$ $mL.s^{-1}$, preferably from $0.001$ $mL.s^{-1}$ to $20$ $mL.s^{-1}$, more preferably from $0.001$ $mL.s^{-1}$ to $10$ $mL.s^{-1}$, even more preferably from $0.001$ $mL.s^{-1}$ to $0.2$ $mL.s^{-1}$, better from $0.001$ $mL.s^{-1}$ to $0.05$ $mL.s^{-1}$, still better of about $0.01$ $mL.s^{-1}$. Advantageously, the aqueous phase is added dropwise to the oil phase at a rate of about $0.001$ $mL.s^{-1}$, $0.007$ $mL.s^{-1}$, $0.01$ $mL.s^{-1}$, $0.1$ $mL.s^{-1}$, $1$ $mL.s^{-1}$, $1.5$ $mL.s^{-1}$, $2$ $mL.s^{-1}$, $3$ $mL.s^{-1}$, $4$ $mL.s^{-1}$, $5$ $mL.s^{-1}$, $6$ $mL.s^{-1}$, $7$ $mL.s^{-1}$, $8$ $mL.s^{-1}$, $9$ $mL.s^{-1}$, $10$ $mL.s^{-1}$, $15$ $mL.s^{-1}$, $20$ $mL.s^{-1}$, $25$ $mL.s^{-1}$, $30$ $mL.s^{-1}$, $35$ $mL.s^{-1}$,

40 mL.s$^{-1}$, 45 mL.s$^{-1}$, or 50 mL.s$^{-1}$.

During the method according to the invention for the manufacture of a water-in-oil-in-water double emulsion, the aqueous phase is advantageously at a temperature ranging from 0°C to 100°C, preferably from 10°C to 80°C, more preferably from 15°C to 60°C. During the method according to the invention for the manufacture of a water-in-oil-in-water double emulsion, the oil phase is advantageously at a temperature ranging from 0°C to 100°C, preferably from 10°C to 80°C, more preferably from 15°C to 60°C.

Advantageously, the mixture comprising the aqueous phase and the oil phase is continuously stirred throughout addition of the aqueous phase, preferably at a stirring speed ranging from 100 rpm to 3000 rpm, more preferably at a stirring speed ranging from 500 rpm to 2500 rpm, even more preferably at a stirring speed ranging from 500 rpm to 2000 rpm.

Advantageously, the stirring allows to emulsify the mixture of the oil phase and aqueous phase.

Advantageously, when drops of the aqueous phase come into contact with the oil phase under agitation, drops of the aqueous phase are dispersed, still in the form of drops, known as single drops.

Advantageously, the stirring is preferably a mechanical stirring.

Advantageously, any type of stirrer commonly used to form emulsions can be used, such as a mechanical paddle stirrer, a static emulsifier, an ultrasonic homogenizer, a membrane homogenizer, a high-pressure homogenizer, a colloid mill, a high-shear disperser or a high-speed homogenizer.

Advantageously, the stirring of the mixture comprising the aqueous phase and the oil phase may be performed in a stirrer. Said stirrer may be selected from the group consisting of overhead mixer equipped with blades, vortex device, static mixer and rotary mixer. Preferably, said stirrer is an anchor mixer (i.e. an overhead mixer equipped with anchor).

More advantageously, the blades of the overhead mixers equipped with blades are selected from the group consisting of helicoidal paddles, sawtooth blade, cross-blade, straight-blade, pitched blade, ringed blades, anchor, propeller, radial flow, cross paddle, centrifugal paddle, half-moon paddle, coil paddle, beater paddle, chain paddle and any combinations thereof.

More advantageously, the vortex device may be a high capacity tube rack vortex mixer, preferably a high capacity tube rack vortex mixer that is orbital, vertical or horizontal.

More advantageously, the static mixer is selected from the group consisting of helical static mixers, plate-like static mixers, low pressure drop static mixers, and Interfacial Surface Generator mixers.

More advantageously, the rotary mixer is selected from the group consisting of planetary mixers, orbital mixers including tank mixers for industrial scale production, and Couette mixers (as described in FR 9604736).

Advantageously, the shear rate applied during stirring ranges from 1000 s$^{-1}$ to 4000 s$^{-1}$, preferably from 2000 s$^{-1}$ to 3000 s$^{-1}$.

Advantageously, the shear rate applied during stirring ranges from 10 s$^{-1}$ to 1000 s$^{-1}$, preferably from 100 s$^{-1}$ to 1000 s$^{-1}$.

The stirring of the aqueous phase and oil phase is stopped when the catastrophic inversion occurs (i.e. when φmax is achieved). When φmax is achieved, a phase inversion occurs in the emulsion and a double emulsion is obtained.

Dynamic viscosity

Advantageously, the ratio between the dynamic viscosity of the aqueous phase and the dynamic viscosity of the oil phase ranges from 0.01 to 20, preferably from 0.01 to 10, more preferably from 0.1 to 10, even more preferably from 1 to 10.

Indeed, the inventors have surprisingly discovered that the formation of a W/O single emulsion with a high incorporation of aqueous phase, also named high internal phase emulsion (which is measured by an elevated cpmax, preferably a φmax equal to or greater than 40% w/w relative to the total weight of the W/O emulsion) is favored by having a low dynamic viscosity ratio p, in particular a ratio p ranging from 0.01 to 20, preferably from 0.1 to 10, more preferably from 1 to 10, even more preferably from 1 to 5.

Regardless of the viscosity or chemical properties of the active agent(s) optionally present in the aqueous phase or its, a dynamic viscosity ratio p ranging from about 1 to 10 allows a significantly slow destabilization kinetics of the emulsion drops, for example allowing the polymerization of microcapsules before the emulsion destabilizes.

Advantageously, the relatively high viscosity of the oil phase contributes to the stability of the single emulsion and contributes to the obtainment of a double emulsion during the process according to the invention for manufacturing double emulsion.

Adhesion energy

The inventors of the present invention have surprisingly discovered that, in surfactant-free systems, adhesion between two droplets of aqueous phase dispersed in the oil phase is necessary and sufficient to assure a double emulsion. The metastability of surfactant-free inverse emulsions is therefore correlated with adhesion. Adhesion has been observed for different oil families, making this phenomenon non-specific to a given system.

Advantageously, the adhesion energy between two droplets of aqueous phase dispersed in the oil phase ranges from $10^{-5}$ J.m$^{-2}$ to $10^{-3}$ J.m$^{-2}$.

Composition of the oil phase

Advantageously, the oil phase comprises at least one compound selected from oligomers, monomers and mixtures thereof, and optionally at least one photo initiator. More advantageously, the oil phase comprises at least one monomer or oligomer, at least one cross-linking agent and at least one photoinitiator. Advantageously, the phase oil further comprises at least one oil selected from the group consisting of silicone oils, mineral oils and mixtures thereof. More advantageously, the silicone oils may be selected from the group consisting of poly(dimethylsiloxane) (PDMS), poly(monomethylsiloxane) (PPMS), poly(dimethylsiloxane) hydroxy terminated (PDMS-OH) and mixtures thereof. More advantageously, the mineral oils may be selected from the group consisting of soybean oil, sunflower oil, jojoba oil, coconut oil, lanolin oil, castor oil, derivatives thereof and mixtures thereof.

Advantageously, the phase oil comprises at least one oil selected from the group consisting of silicone oils, mineral oils and mixtures thereof. More advantageously, the silicone oils may be selected from the group consisting of poly(dimethylsiloxane) (PDMS), poly(monomethylsiloxane) (PPMS), poly(dimethylsiloxane) hydroxy terminated (PDMS-OH) and mixtures thereof. More advantageously, the mineral oils may be selected from the group consisting of soybean oil, sunflower oil, jojoba oil, coconut oil, lanolin oil, castor oil, derivatives thereof and mixtures thereof.

Even more advantageously, the phase oil comprises a mixture of poly(dimethylsiloxane) (PDMS), poly(monomethylsiloxane) (PPMS), and poly(dimethylsiloxane) hydroxy terminated (PDMS-OH).

According to an embodiment, the phase oil consists of PDMS-OH. Indeed, a phase oil consisting of PDMS-OH has been found to be extremely efficient with a cpmax greater than 90%, for example in an aqueous/oil system being glycerol (1 Pa.s) / PDMS-OH (from 1 to 10 Pa.s).

According to another embodiment, the oil phase is a mixture comprising hexadecane and PDMS. More advantageously, the oil phase is a mixture comprising hexadecane and PDMS, wherein the mass fraction of hexadecane is of up to 75% w/w relative to the mass of the hexadecane/PDMS mixture.

Advantageously, the oil phase is a crosslinkable composition. More advantageously, the oil phase is a photocrosslinkable composition. In other words, the oil phase may further comprise a crosslinking agent, preferably a photocrosslinking agent.

Advantageously, the viscosity of the oil phase at 25°C and at atmospheric pressure ranges from 50 mPa.s to 500,000 mPa.s, preferably from 500 mPa.s to 100 000 mPa.s. More preferably, the viscosity of the oil phase at 25°C and atmospheric pressure ranges from 1,000 mPa.s to 50,000 mPa.s, even more preferably from 2,000 mPa.s to 25,000 mPa.s, and for example from 3,000 mPa.s to 15,000 mPa.s.

Advantageously, the oil phase further comprises at least one monomer or oligomer, at least one cross-linking agent and at least one initiator. More advantageously, the oil phase further comprises at least one monomer or oligomer, at least one cross-linking agent and at least one photoinitiator.

More advantageously, the oil phase may comprise from 50% to 99% by weight of monomer(s) and/or oligomer(s), relative to the total weight of the oil phase.

More advantageously, the oil phase may comprise from 1% to 20% by weight of at least one crosslinking agent, relative to the total weight of the oil phase.

More advantageously, the oil phase may comprise from 0.1% to 5% by weight of at least one initiator, relative to the total weight of the oil phase.

More advantageously, the oil phase may comprise from 0.1% to 5% by weight of at least one photoinitiator, relative to the total weight of the oil phase.

Advantageously, the oil phase may further comprise at least one additional monomer or oligomer, in particular at least one additional monomer or oligomer capable of improving the properties of the microcapsule and/or imparting new properties to the microcapsule. The at least one additional monomer or oligomer are different from the at least one monomer or oligomer. Thus, when the oil phase further comprises at least one monomer or oligomer and at least one additional monomer or oligomer, then the oil phase comprises at least two monomers or oligomers different from each other. More advantageously, the oil phase may further comprise at least one additional monomer or oligomer selected from the group consisting of silanes and alcohol acrylates. Indeed, silanes and alcohol acrylates contribute to optimize the phase inversion of the emulsion during the method according to the present invention for manufacturing a double emulsion.

Composition of the aqueous phase

Advantageously, the aqueous phase comprises at least one active agent.

More advantageously, the aqueous phase comprises from 1% to 99% by weight, preferably from 5% to 95% by weight, more preferably from 10% to 90% by weight, even more preferably from 20% to 80% by weight, better from 30% to 70% by weight, still better from 40% to 60% by weight, of at least one active agent, relative to the total weight of the aqueous phase.

More advantageously, the aqueous phase is a monophasic liquid composition, meaning that the at least one active agent is in a pure form or is solubilized into the aqueous phase.

W/O systems exhibiting particular metastability after phase inversion to W/O/W emulsion

According to a particular embodiment, the oil phase comprises a silicone oil having a viscosity ranging from 0.05 to 30 Pa.s and the aqueous phase is selected from the group consisting of glycerol, a glycerol aqueous solution, an alginate aqueous solution and mixtures thereof.

According to another particular embodiment, the oil phase comprises a polyacrylate having a viscosity ranging from 1 to 25 Pa.s and the aqueous phase is selected from the group consisting of glycerol and a glycerol aqueous solution.

According to another particular embodiment, the oil phase comprises a polyurethane having a viscosity of 10 Pa.s and the aqueous phase is selected from the group consisting of glycerol and a glycerol aqueous solution.

According to another particular embodiment, the oil phase comprises a polyester having a viscosity of 2 Pa.s and the aqueous phase is selected from the group consisting of glycerol and a glycerol aqueous solution.

Indeed, the inventors of the present application have surprisingly discovered that these four W/O systems exhibit adhesion, the adhesion energy between two droplets of aqueous phase dispersed in the oil phase ranging from $10^{-5}$ J.m$^{-2}$ to $10^{-3}$ J.m$^{-2}$, and allows the obtainment of a W/O/W double emulsion after phase inversion with a particular metastability.

**Double emulsion obtained by the method according to the invention.**

The present invention also relates to a water-in-oil-in-water double emulsion obtained according to the method according to the invention for the manufacture of a water-in-oil-in-water double emulsion.

Advantageously, all the features and advantageous features described in part "Method for preparing a double emulsion", subpart "Method for preparing a double emulsion" of the present application, apply *mutatis mutandis* for the water-in-oil-in-water double emulsion obtained according to the method according to the invention for the manufacture of a water-in-oil-in-water double emulsion.

Advantageously, the morphology of the water-in-oil-in-water double emulsion is process-dependent: it depends notably on the shear rate, on the stirring speed, on the internal phase addition speed, on the adhesion energy, on the interfacial tension $\gamma$ between the aqueous phase and the oil phase, on the viscosity ratio p, on the composition of the aqueous phase and on the composition of the oil phase.

**Double emulsion**

The present invention also relates to a water-in-oil-in-water double emulsion, comprising two aqueous phases (an innermost and an external aqueous phases) and one oil phase, wherein one of the two aqueous phases (the innermost aqueous phase) is entrapped in the oil phase and the second aqueous phase (the external aqueous phase) entraps the oil phase, wherein the two aqueous phases have the same composition, wherein the adhesion energy between two droplets of the innermost aqueous phase dispersed in the oil phase ranges from $10^{-5}$ J.m$^{-2}$ to $10^{-3}$ J.m$^{-2}$.

Advantageously, the contact angle ranges from 1 to 30 degrees, preferably more preferably from 10 to 30 degrees, more preferably from 20 to 30 degrees, as determined from a film of the oil phase polymer onto which the innermost aqueous phase is dispersed.

Advantageously, the interfacial tension $\gamma$ between the aqueous phase and the oil phase ranges from 0 J.m$^{-2}$ to $50 \times 10^{-3}$ J.m$^{-2}$, preferably from $20 \times 10^{-3}$ J.m$^{-2}$ to $40 \times 10^{-3}$ J.m$^{-2}$. Advantageously, such a low interfacial tension between the aqueous phase and the oil phase advantageously ensures the stability of the W/O/W double emulsion according to the invention.

Advantageously, the weight ratio of innermost aqueous phase to oil phase ranges from 0 to 100, preferably from 20 to 70, more preferably from 20 to 60.

Advantageously, the ratio between the dynamic viscosity of the innermost aqueous phase and the viscosity of the oil phase ranges from 0.01 to 20, preferably from 0.01 to 10, more preferably from 0.1 to 10, even more preferably from 1 to 10.

Regardless of the viscosity or chemical properties of the active agent(s) optionally present in the aqueous phases, a dynamic viscosity ratio p ranging from about 1 to 10 allows a significantly slow destabilization kinetics of the emulsion drops, for example allowing the polymerization of microcapsules before the emulsion destabilizes.

Thus, the relatively high viscosity of the oil phase ensures the stability of the single emulsion and contributes to the obtainment of a double emulsion during the process according to the invention for manufacturing double emulsion.

Advantageously, all the features and advantageous features of the oil phase described in part "Method for preparing a double emulsion", subpart "Composition of the oil phase" of the present application, apply *mutatis mutandis* for the oil phase of the double emulsion.

Advantageously, all the features and advantageous features of the aqueous phase and optional active agent described in part "Method for preparing a double emulsion", subpart "Composition of the aqueous phase" of the present application, apply *mutatis mutandis* for the aqueous phases and optional active agent of the double emulsion.

Advantageously, all the features and advantageous features of the W/O system described in part "Method for preparing a double emulsion", subpart "W/O systems exhibiting particular metastability after phase inversion to W/O/W emulsion" of the present application, apply *mutatis mutandis* for the W/O system of the double emulsion.

The W/O/W emulsion according to the invention exhibits a particular metastability and allows a high internal phase ratio (cpmax equal or greater than 50% w/w).

## Use of the double emulsion

The present invention also relates to the use of the water-in-oil-in-water double emulsion according to the invention for encapsulating at least one hydrophilic active agent.

The present invention also relates to the use of the water-in-oil-in-water double emulsion in at least one field selected from the group consisting of agriculture, cosmetics, home care, chemical, agrochemical, paint, fuel, lubricant, bitumen, and drilling sludges or muds field.

Advantageously, the water-in-oil-in-water double emulsion according to the invention is used in the agrochemical field. Advantageously, the double emulsion is used in a composition for agrochemicals.

The present invention also relates to a composition for agrochemicals comprising the water-in-oil-in-water double emulsion according to the invention.

Advantageously, the water-in-oil-in-water double emulsion according to the invention is used in any field other than cosmetic. More advantageously, the water-in-oil-in-water double emulsion according to the invention is used in at least one field selected from the group consisting of agriculture, home care, chemical, agrochemical, paint, fuel, lubricant, bitumen, and drilling sludges or muds field.

## Method for preparing solid microcapsules

The invention also relates to a method for preparing solid microcapsules comprising the steps of:

    a) manufacturing a water-in-oil-in-water double emulsion according to the method according to the invention, wherein the oil phase comprises at least one compound selected from oligomers, monomers and mixtures thereof and optionally at least one photo initiator, and then
    b) crosslinking the oligomers and/or monomers of the oil phase.

Thus, advantageously, the middle phase of the double emulsion obtained in step a) is then solidified in step b) to manufacture solid microcapsules.

Advantageously, the aqueous phase comprises at least one active agent. In this case, the method according to the invention for preparing solid microcapsules ensures the storage and protection of the active ingredients of interest.

Advantageously, the method according to the invention for preparing solid microcapsules is optimally suited for hydrophilic active agents, avoids the use of surfactants, and comprises only one step to obtain the double emulsion to be polymerized.

Advantageously, the step b) of crosslinking the oligomers and/or monomers of the oil phase is carried out by photopolymerization, more advantageously, it is a UV cross-linking of the oil phase. This photopolymerization step allows in particular to solidify the middle layer of the double emulsion and thus eliminates any coalescence.

Advantageously, step b) makes it possible to obtain microcapsules encapsulating the aqueous phase. More advantageously, if the aqueous phase comprises at least one active agent, step b) makes it possible to obtain microcapsules encapsulating the aqueous phase comprising the at least one active agent and thus encapsulating also the at least one active agent.

Advantageously, step b) consists in exposing the double emulsion obtained in step a) to a light source capable of initiating the photopolymerization of the oil phase. Preferably, the light source is a UV light source. More preferably, the UV light source may emit in the wavelength ranging from 100 nm to 400 nm.

Advantageously, during step b), the oil phase of the emulsion obtained in step a) is exposed to a light source for a period of less than 15 minutes, preferably for a period ranging from 10 seconds to 10 minutes, more preferably for a period ranging from 20 seconds to 200 seconds, even more preferably for a period ranging from 20 seconds to 180 seconds, better for a period ranging from 30 seconds to 50 seconds, even better for a period of about 30 seconds.

Advantageously, during step b), the shell of the double drops, consisting of photocrosslinkable oil phase, is crosslinked, encapsulating and protecting the aqueous phase, optionally comprising at least one ingredient, from being released in the absence of a trigger, in particular a mechanical trigger.

Advantageously, the composition obtained at the end of step b), comprising solid microcapsules dispersed in the external aqueous phase, is ready for use and can be used without any additional capsule post-processing step being required.

Advantageously, the method according to the invention for preparing solid microcapsules may be a continuous or a batch method for preparing solid microcapsules. More advantageously, the method of the invention is a batch method.

The method according to the invention for preparing solid microcapsules has the advantage that no surfactant is required for manufacturing solid microcapsules.

The crosslinking of step b), once complete, then provides thermodynamic stabilization of the double emulsion.

## Capsule

The invention also relates to a solid microcapsule obtained by the method according to the invention for preparing solid microcapsules.

The invention also relates to a solid microcapsule comprising (i) a polymer that is crosslinked in three dimensions forming a polymer network, the polymer network delimiting the capsule shell and creating a matrix in the core of the capsule, and (ii) drops of an aqueous phase entrapped in the polymer matrix in the core of the capsule.

Advantageously, the aqueous phase comprises at least one active agent, ie. an active ingredient of interest. In this case, the solid microcapsule according to the invention ensures the storage and protection of the active ingredients of interest. Advantageously, the active agent may be released from the solid microcapsule by mechanical disruption of the polymer phase or by passive diffusion.

More advantageously, the solubility of the hydrophile active agent in the aqueous phase is equal to or greater than 20 g.L$^{-1}$.

Advantageously, the solid microcapsule according to the invention corresponds to a collection of multicore capsules in which multiple droplets of the aqueous phase are embedded within the polymer network.

Advantageously, the polymer network is an oil phase that is cross-linked in three dimensions. More advantageously, the polymer chains are not only on the shell but also inside the capsule, forming a bond or a matrix between the droplets of aqueous phase. This type of structure (a network of polymer(s) with aqueous inclusions) is an optimal configuration for allowing encapsulation of hydrophilic active agents.

Advantageously, the crosslinked polymer is selected from the group consisting of crosslinked polyesters, crosslinked polyepoxides, and crosslinked polyurethanes.

According to one embodiment, there is a concentration gradient of the at least one active agent in the aqueous droplet, wherein the concentration is greater at the center of the aqueous droplet than at the periphery of the aqueous droplet. More advantageously, the concentration of active agent in the polymer network is of less than 10% w/w, preferably less than 1% w/w, more preferably less than 0.1% w/w, relative to the total weight of the active agent in the microcapsule.

Advantageously, the mean diameter of the solid microcapsule ranges from 0.1 $\mu$m to 20 $\mu$m, preferably from 10 $\mu$m to 20 $\mu$m.

Advantageously, the inclusions of the aqueous phase are in the form of droplets, preferably in the form of droplets having a mean diameter ranging from 1 $\mu$m to 2 $\mu$m.

## Use of the capsule

The present invention also relates to the use of at least one microcapsule according to the invention for encapsulating at least one hydrophilic active agent.

The present invention also relates to the use of at least one microcapsule according to the invention in at least one field selected from the group consisting of agriculture, cosmetics, home care, chemical, agrochemical, paint, fuel, lubricant, bitumen, and drilling sludges or muds field.

Advantageously, the microcapsule according to the invention is used in cosmetics. Advantageously, the microcapsule according to the invention is used in a cosmetic composition.

The present invention also relates to a cosmetic composition comprising at least one microcapsule according to the invention.

Advantageously, the microcapsule according to the invention is used in any field other than cosmetics. More advantageously, the microcapsule according to the invention is used in at least one field selected from the group consisting of agriculture, home care, chemical, agrochemical, paint, fuel, lubricant, bitumen, and drilling sludges or muds field.

**BRIEF DESCRIPTION OF THE DRAWINGS**

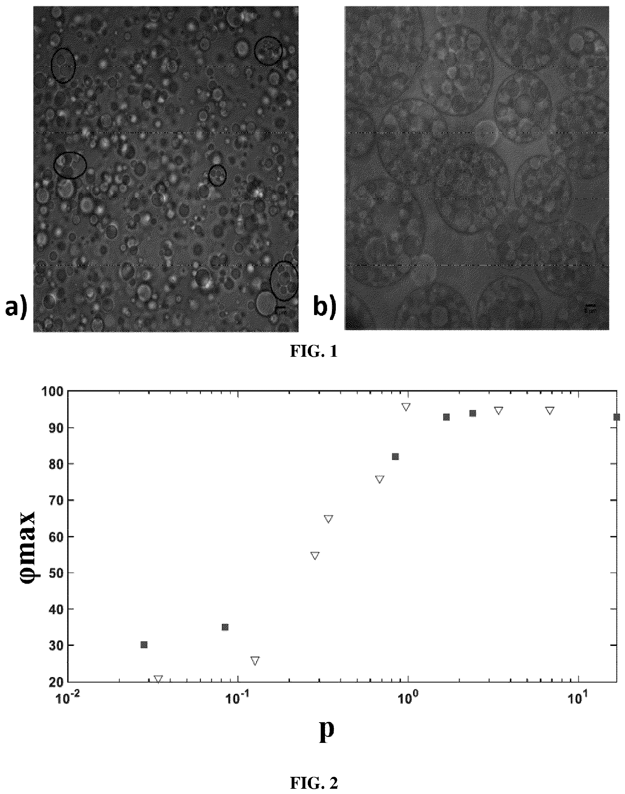

**Figure 1** represents two pictures from microscopic observation of an emulsion before and after phase inversion, whose water/oil system is glycerol (1 Pa.s) / silicone (1 Pa.s). Figure 1a) illustrates the emulsion before inversion ($\varphi < c_{pmax}$) and Figure 1b) illustrates the emulsion after inversion ($\varphi = \varphi_{max}$).

**Figure 2** is a graph showing the evolution of $\varphi_{max}$ depending on the viscosity ratio p for two different water/oil systems: one with PDMS as oil phase and pure glycerol as aqueous phase (curve represented by solid squares), the other with PDMS as oil phase and a 95% (w/w) glycerol solution as aqueous phase (curve represented by empty triangles).

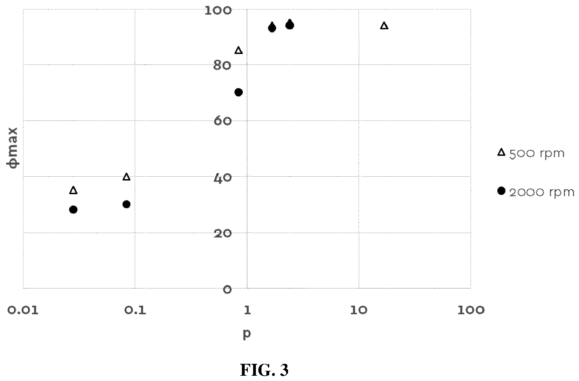

**Figure 3** is a graph showing the evolution of $\varphi_{max}$ depending on the viscosity ratio p at two stirring rates (500 rpm and 2000 rpm) for a water/oil system comprising glycerol as aqueous phase and silicone oil as oil phase.

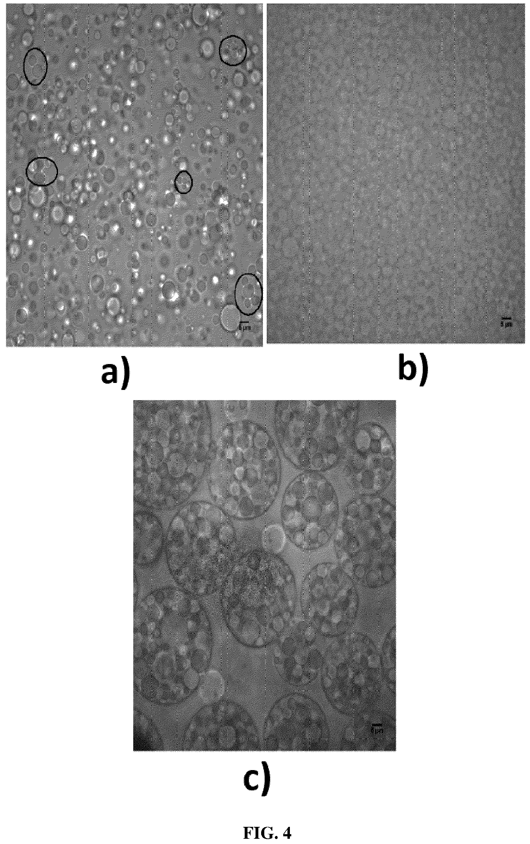

**Figure 4** represents three pictures from microscopic observation of an emulsion showing the evolution of $\varphi$ until $\varphi_{max}$ is achieved, the water/oil system comprising glycerol as aqueous phase and silicone oil as oil phase and having a viscosity ratio p of about 1, the stirring speed being of 500 rpm. In **Figure 4a)**, $\varphi$ = 10% w/w. In **Figure 4b)**, $\varphi$ = 75% w/w. In **Figure 4c)**, $\varphi$ = 85% w/w.

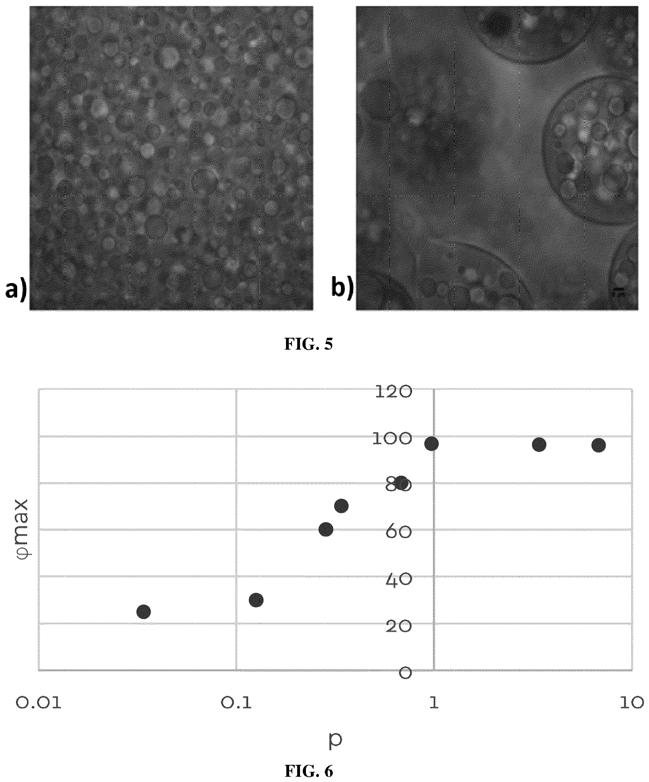

**Figure 5** represents two pictures from microscopic observation of an emulsion showing the evolution of $\varphi$ until $\varphi_{max}$ is achieved, the water/oil system comprising glycerol as aqueous phase and silicone oil as oil phase and having a viscosity ratio of about 0.1, the stirring speed being of 500 rpm. In **Figure 5a)**, $\varphi$ = 20% w/w. In **Figure 5b)**, $\varphi$ = 40% w/w.

**Figure 6** is a graph showing the evolution of $\varphi_{max}$ depending on the viscosity ratio p at a stirring rate of 500 rpm for a water/oil system comprising a 95% (w/w) aqueous glycerol solution as aqueous phase and silicone oil as oil phase.

**Figure 7** is a graph showing the evolution of $\varphi_{max}$ depending on the viscosity ratio p at a stirring rate of 500 rpm for a water/oil system comprising a PEG20000 aqueous solution (26% w/w) (0.1 Pa.s) as aqueous phase and silicone oil as oil phase.

**Figure 8** is schematic representation of the method of determining the adhesion between two drops comprising the use of a micropipette aspiration for adhesion testing.

**EXAMPLES**

The present invention is further illustrated by the following examples.

**Example 1: Impact of the adhesion energy between two droplets of aqueous phase dispersed in the oil phase on inverse emulsions formulated**

**Materials and Methods**

Different water/oil systems were studied. Each system has a given dynamic viscosity ratio p which is about 1. For each system, no surfactant was added.
In order to obtain an inverse emulsion, for each water/oil system, the aqueous phase was slowly added, using a syringe, to a given starting volume of the oil phase in a container comprising an overhead stirrer with an anchor geometry (the stirrer diameter being 45 mm, stirring speed being 500 rpm), at a temperature of 25°C.
After each addition step of the aqueous phase, an aliquot of the water/oil mixture was taken and observed under the microscope. For each system, $c_{pmax}$ was determined and the morphology of the inverse emulsion was checked.

**Results**

The results are presented in the following **Table 1:**

Table 1:

| Oil phase | Aqueous phase | Surface tension (mN/m) | Adhesion energy between two droplets of aqueous phase dispersed in the oil phase ranging from $10^{-5}$ J.m$^{-2}$ to $10^{-3}$ J.m$^{-2}$ | Phase inversion |
|---|---|---|---|---|
| Silicone oil (equivalent to PDMS) (from 0.1 Pa.s to 30 Pa.s) | Glycerol (1 Pa.s) | 25.3 | Yes: $3.1\times10^{-5}$ J.m$^{-2}$ | Double emulsion |
| Silicone oil (equivalent to PDMS) (from 0.1 Pa.s to 30 Pa.s) | PEG20000 aqueous solution (26% w/w) (0.1 Pa.s) | - | Yes: $1.4\times10^{-5}$ J.m$^{-2}$ | Double emulsion |
| Silicone oil (equivalent to PDMS) (from 0.1 Pa.s to 30 Pa.s) | Alginate aqueous solution (1 Pa.s) | 40 | Yes: $1.4\times10^{-5}$ J.m$^{-2}$ | Double emulsion |
| Mineral oil (1 Pa.s) | Glycerol (1 Pa.s) | 28.5 | No* | Simple emulsion |
| * The value of adhesion energy between the two droplets of aqueous phase dispersed in the oil phase for the system glycerol (1 Pa.s) / mineral oil (1 Pa.s) could not be determined because the system was not stable, so there was no double drop and the continuous film method was not applicable. | | | | |

As it can be seen in **Table 1,** there is a relation between the adhesion energy between two droplets of aqueous phase dispersed in the oil phase, and the type of phase inversion. Indeed, when the adhesion energy between two droplets of aqueous phase dispersed in the oil phase ranges from $10^{-5}$ J.m$^{-2}$ to $10^{-3}$ J.m$^{-2}$, the phase inversion leads to the obtainment of a double emulsion, whereas when the adhesion energy between two droplets of aqueous phase dispersed in the oil phase does not range from $10^{-5}$ J.m$^{-2}$ to $10^{-3}$ J.m$^{-2}$, the phase inversion leads to the obtainment of a simple emulsion.

In addition, **Figure 1** illustrates that, starting from a glycerol-in-silicone emulsion in which the viscosity ratio p is about 1 (p$\approx$1) **(Figure 1a),** $\varphi < \varphi$max), by reaching cpmax, the emulsion does not phase invert into a simple emulsion as expected but turned into a double emulsion of glycerol/silicone/glycerol **(Figure 1b),** $\varphi = \varphi$max).

**Example 2: Influence of the dynamic viscosity ratio on inverse emulsions formulated**

**Materials and Methods**

Three different water/oil systems were studied. For each system, no surfactant was added.

In order to obtain inverse emulsions, for each water/oil system, the aqueous phase was slowly added, using a syringe, to a given starting volume of the oil phase in a container comprising an overhead stirrer with an anchor geometry (the stirrer diameter being 45 mm, stirring speed being 500 rpm), at a temperature of 25°C.

After each addition step of the aqueous phase, an aliquot of the water/oil mixture was taken and observed under the microscope. For each system, cpmax was determined and the morphology of the inverse emulsion was checked.

**Results**

The results are presented in the following **Table 2** and in **Figures 2 to 7.**

**Table 2:**

| System number | Oil phase | Aqueous phase | Adhesion energy between two droplets of aqueous phase dispersed in the oil phase ranging from $10^{-5}$ J.m$^{-2}$ to $10^{-3}$ J.m$^{-2}$ | Phase inversion |
|---|---|---|---|---|
| 1 | Silicone oil (equivalent to PDMS) (0.1 Pa.s to 30 Pa.s) | Glycerol (1 Pa.s) | Yes: $3.1 \times 10^{-5}$ J.m$^{-2}$ | Double emulsion |
| 2 | Silicone oil (equivalent to PDMS) (0.1 Pa.s to 30 Pa.s) | 95% (w/w) aqueous glycerol solution (350 mPa.s) | Yes: $3.1 \times 10^{-5}$ J.m$^{-2}$ | Double emulsion |
| 3 | Silicone oil (equivalent to PDMS) (0.1 Pa.s to 30 Pa.s) | PEG20000 aqueous solution (26% w/w) (0.1 Pa.s) | Yes: $1.4 \times 10^{-5}$ J.m$^{-2}$ | Double emulsion |

Systems number 1 (glycerol / silicone oil (0.1 Pa.s to 30 Pa.s)): as it can be seen in **Figure 2, Figure 3, Figure 4 and Figure 5,** it was noticed that $\varphi$max highly depends on the viscosity ratio p. Elevated $\varphi$max (i.e. of equal to or greater than 64% w/w) is favored when p is in the order of 1 or greater than or equal to 1. For p being about 3, $\varphi$max is equal to 95% w/w. When $\varphi$max is equal to 95% w/w, the obtained double emulsion showed a remarkable metastability of the internal emulsion which lasted at least one day. As it can be seen in **Figure 2,** at low dynamic viscosity ratio (p<1), when the outer oil phase is more viscous than the inner aqueous phase, the resulting inverting double emulsion could not be concentrated to more than 70% w/w in innermost aqueous phase ($\varphi$<70% w/w). By increasing the viscosity ratio, more innermost aqueous phase could be incorporated in the intermediate oil phase. In particular, for p>1, $\varphi$ could reach 90% w/w, which is surprising. In addition, it can be seen in **Figure 3** that cpmax also depends on the rotation speed.

For system numbers 2 and 3, as it can be seen respectively in **Figure 6** and **Figure 7,** it was noticed that $\varphi$max highly depends on the viscosity ratio p.

**Claims**

1. A method for the manufacture of a water-in-oil-in-water double emulsion, comprising:

   a step of adding dropwise an aqueous phase to an oil phase, wherein the adhesion energy between two droplets of aqueous phase dispersed in the oil phase ranges from $10^{-5}$ J.m$^{-2}$ to $10^{-3}$ J.m$^{-2}$, said step of adding the aqueous phase to the oil phase being performed until a catastrophic inversion occurs.

2. The method according to claim **1,** wherein the addition dropwise of the aqueous phase to the oil phase occurs at a rate ranging from 0.001 mL.s$^{-1}$ to 50 mL.s$^{-1}$, preferably from 0.001 mL.s$^{-1}$ to 20 mL.s$^{-1}$, more preferably from 0.001 mL.s$^{-1}$ to 10 mL.s$^{-1}$, even more preferably from 0.001 mL.s$^{-1}$ to 0.2 mL.s$^{-1}$, better from 0.001 mL.s$^{-1}$ to 0.05 mL.s$^{-1}$, still better of about 0.01 mL.s$^{-1}$.

3. The method according to claim **1** or **2,** wherein the mixture comprising the aqueous phase and the oil phase is continuously stirred, preferably at a stirring speed ranging from 100 rpm to 3000 rpm, more preferably at a stirring speed ranging from 500 rpm to 2500 rpm.

4. The method according to claim **3,** wherein the shear rate applied during stirring ranges from 1000 s$^{-1}$ to 4000 s$^{-1}$, preferably from 2000 s$^{-1}$ to 3000 s$^{-1}$.

5. A water-in-oil-in-water double emulsion, comprising two aqueous phases (an innermost and an external aqueous phases) and one oil phase, wherein one of the two aqueous phases (the innermost aqueous phase) is entrapped in the oil phase and the second aqueous phase (the external aqueous phase) entraps the oil phase, wherein the two aqueous phases comprise the same composition, wherein the adhesion energy between two droplets of the innermost aqueous phase dispersed in the oil phase ranges from $10^{-5}$ J.m$^{-2}$ to $10^{-3}$ J.m$^{-2}$.

6. The method according to any one of claims **1** to **4,** or the water-in-oil-in-water double emulsion according to claim **5,** wherein said double emulsion does not comprise surfactant.

7. The method according to any one of claims **1** to **4** or **6,** or the water-in-oil-in-water double emulsion according to claim **5** or **6,** wherein the ratio between the dynamic viscosity of the innermost aqueous phase and the viscosity of the oil phase ranges from 0.01 to 20, preferably from 0.01 to 10, more preferably from 0.1 to 10, even more preferably from 1 to 10,
   the dynamic viscosities being measured using a TA Instruments AR-G2 rheometer equipped with a 3 cm diameter, 2-degree angle cone and a temperature control cell set at 25°C.

8. The method according to any one of claims **1** to **4** or **6** or **7,** or the water-in-oil-in-water double emulsion according to any one of claims **5** to **7,** wherein the interfacial tension $\gamma$ between the aqueous phase and the oil phase ranges from 0 J.m$^{-2}$ to $50 \times 10^{-3}$ J.m$^{-2}$, preferably from $20 \times 10^{-3}$ J.m$^{-2}$ to $40 \times 10^{-3}$ J.m$^{-2}$.

9. The method according to any one of claims **1** to **4** or **6** to **8,** or the water-in-oil-in-water double emulsion according to any one of claims **5** to **8,** wherein the oil phase comprises at least one compound selected from oligomers, monomers and mixtures thereof, and optionally at least one photo initiator.

10. A water-in-oil-in-water double emulsion obtained by the method according to any one of claims **1** to **4** or **6** to **9.**

11. A method for preparing solid microcapsules comprising the steps of: a) manufacturing a water-in-oil-in-water double emulsion according to the method according to any one of claims **1** to **4** or **6** to **9,** wherein the oil phase comprises at least one compound selected from oligomers, monomers and mixtures thereof and optionally at least one photo initiator, and then b) crosslinking the oligomers and/or monomers of the oil phase.

12. The method according to claim **11,** wherein the step b) of crosslinking is carried out by submitting the double emulsion to a source of light, preferably a source of UV light.

13. A solid microcapsule obtained by the method according to claim **11** or **12.**

14. A solid microcapsule comprising:

    - a polymer that is crosslinked in three dimensions forming a polymer network, the polymer network delimiting the capsule shell and creating a matrix in the core of the capsule, and
    - drops of an aqueous phase entrapped in the polymer matrix in the core of the capsule.

15. The solid microcapsule according to claim **14,** wherein the aqueous phase comprises at least one hydrophile active agent,
    preferably said solid microcapsule comprises greater than or equal to 20% by weight, more preferably from 20% to 80% by weight, even more preferably from 40% to 60% by weight, better about 50% by weight, of at least one hydrophile active agent, the weight percentages being expressed relative to the total weight of said solid microcapsule.

16. The solid microcapsule according to any one of claims **14** to **15,** wherein the ratio of the weight of the entrapped aqueous phase to the weight of the polymer network ranges from 20 to 70.

FIG. 1

FIG. 2

**FIG. 3**

a)

b)

c)

FIG. 4

**FIG. 5**

**FIG. 6**

**FIG. 7**

**FIG. 8**

Europäisches
Patentamt

European
Patent Office

Office européen
des brevets

# EUROPEAN SEARCH REPORT

Application Number

EP 22 30 5808

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/065712 A1 (DOW CORNING [US]; BARNES KATHLEEN [US] ET AL.) 10 June 2010 (2010-06-10) * claim 1 * | 1-12 | INV. B01J13/14 B01J13/18 A01N25/28 A23P10/30 A61K8/11 A61K9/50 C09B67/02 C11D3/50 F28D20/02 |
| A | US 4 626 443 A (TAKAHASHI YASUYUKI [JP] ET AL) 2 December 1986 (1986-12-02) * the whole document * | 1-12 | |
| A | US 4 714 566 A (TAKAHASHI YASUYUKI [JP] ET AL) 22 December 1987 (1987-12-22) * the whole document * | 1-12 | |
| A | DE 41 36 699 A1 (LANCASTER GROUP AG [DE]) 22 October 1992 (1992-10-22) * the whole document * | 1-12 | |

TECHNICAL FIELDS
SEARCHED (IPC)

B01J
A01N
C09B
C11D
A23P
A61K
A61Q
F28F
F28D

The present search report has been drawn up for all claims

| Place of search | Date of completion of the search | Examiner |
|---|---|---|
| Munich | 17 November 2022 | Tarallo, Anthony |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-12

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

# LACK OF UNITY OF INVENTION
## SHEET B

**Application Number**

EP 22 30 5808

The Search Division considers that the present European patent application does not comply with the requirements of unity of invention and relates to several inventions or groups of inventions, namely:

      **1. claims: 1-12**

            **1. A method for the manufacture of a water-in-oil-in-water double emulsion.**
            **5. A water-in-oil-in-water double emulsion.**
            **10. A water-in-oil-in-water double emulsion.**
                    ---


      **2. claims: 13-16**

            **13. A solid microcapsule.**
            **14. A solid microcapsule.**
                    ---

## ANNEX TO THE EUROPEAN SEARCH REPORT
## ON EUROPEAN PATENT APPLICATION NO.

EP 22 30 5808

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2022

| Patent document cited in search report | | Publication date | Patent family member(s) | | Publication date |
|---|---|---|---|---|---|
| WO 2010065712 | A1 | 10-06-2010 | EP | 2367885 A1 | 28-09-2011 |
| | | | US | 2011245374 A1 | 06-10-2011 |
| | | | WO | 2010065712 A1 | 10-06-2010 |
| US 4626443 | A | 02-12-1986 | NONE | | |
| US 4714566 | A | 22-12-1987 | JP | H0338887 B2 | 12-06-1991 |
| | | | JP | S60183031 A | 18-09-1985 |
| | | | US | 4714566 A | 22-12-1987 |
| DE 4136699 | A1 | 22-10-1992 | AT | 158174 T | 15-10-1997 |
| | | | CA | 2082296 A1 | 08-05-1993 |
| | | | DE | 4136699 A1 | 22-10-1992 |
| | | | EP | 0540857 A1 | 12-05-1993 |
| | | | ES | 2109299 T3 | 16-01-1998 |
| | | | HK | 1002724 A1 | 11-09-1998 |
| | | | JP | H05279248 A | 26-10-1993 |
| | | | US | 5478561 A | 26-12-1995 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018172360 A
- US 6335315 B
- US 5877145 A
- FR 9604736

**Non-patent literature cited in the description**

- **LIU et al.** *Polymer Chemistry,* 2013, vol. 4, 3431-3443
- **POULIN et al.** *Phys. Rev. Lett.,* 1996, vol. 77, 3248
- **POULIN et al.** *Phys. Rev. Lett.,* 1997, vol. 79, 4862